# EUROPEAN PATENT SPECIFICATION

(11) **EP 4 086 489 B1**
(45) Date of publication and mention of the grant of the patent: **02.09.2026**
(21) Application number: 19958322.0
(22) Date of filing: 30.12.2019
(51) Int. Cl.: F16K 11/044, A61M 16/01, F16K 31/42

(54) **THREE-WAY VALVE AND ANAESTHESIA MACHINE**
DREIWEGEVENTIL UND ANÄSTHESIEMASCHINE
VANNE À TROIS VOIES ET MACHINE D'ANESTHÉSIE

(43) Date of publication of application: 09.11.2022
(73) Proprietor: Shenzhen Mindray Bio-Medical Electronics Co., Ltd, Shenzhen, Guangdong 518057 (CN)
(72) Inventor: LUO, Caijin, Shenzhen, Guangdong 518057 (CN); WU, Xuetao, Shenzhen, Guangdong 518057 (CN); CHEN, Peitao, Shenzhen, Guangdong 518057 (CN)
(74) Representative: KIPA AB
(86) International application number: PCT/CN2019/130182
(87) International publication number: WO 2021/134349

(56) References cited:
- WO-A1-96/23156
- CN-A- 103 821 965
- CN-A- 103 821 965
- CN-A- 104 482 246
- CN-A- 105 517 614
- CN-A- 105 517 614
- CN-A- 110 220 010
- CN-B- 105 090 559
- CN-U- 203 463 715
- CN-U- 203 463 715
- CN-U- 203 463 715
- CN-U- 207 064 709
- US-A- 4 442 998
- US-A1- 2003 000 586
- US-A1- 2005 076 959

## Description

### TECHNICAL FIELD

The disclosure relates to the technical field of medical devices, and more particularly to a three-way valve and an anaesthesia machine.

### BACKGROUND

The anaesthesia machine has the function of delivering anesthetic drug and maintaining patient ventilation during the operation of patient, and plays vital roles in whether the operation can be carried out normally and in the patient safety. Therefore, the anaesthesia machine needs to carry out various self-inspections before operation. The gas leakage of anaesthesia machine is not only related to the patient safety, but also may have an adverse impact on doctor. Therefore, the leakage self-inspection is a particularly important part in the pre-work self-inspection process of anaesthesia machine.

According to the operation state of the anaesthesia machine, the leakage self-inspection usually includes a leakage self-inspection under a machine-controlled state and a leakage self-inspection human-controlled state. At present, the switch valve of the anaesthesia machine adopts a pure mechanical structure. During the leakage self-inspection, the switch valve needs to be manually controlled to switch the leakage inspection under the human-controlled state and the inspection under the machine-controlled state. The anaesthesia machine cannot complete the leakage inspection fully automatically.

Patent applications US2003000586A1, CN105090559B, CN203463715U, CN103821965A, CN105517614A, US2005076959A1 and US4442998A provide teachings related to the technical field of the application. In particular, CN203463715U discloses a three-way valve having an electric switching mechanism comprising a cylinder drive element in which a piston is located and a pilot solenoid valve configured to connect a gas inlet of the cylinder drive element with a gas source, wherein a valve rod of a valve core assembly of the three-way valve is fixedly connected to the piston of the cylinder drive element.

### SUMMARY

In view of this, an embodiment of this disclosure expects to provide a three-way valve and an anaesthesia machine capable of switching.

To achieve the above purpose, one aspect of an embodiment of this disclosure provides a three-way valve as set forth in claim 1.

Another aspect of an embodiment of this disclosure provides an anaesthesia machine, which includes a controller, a respiratory line, a hand-controlled branch, a machine-controlled branch and the above three-way valve; the first channel is connected with the respiratory line, the second channel is connected with the machine-controlled branch, the third channel is connected with the hand-controlled branch, and the controller is electrically connected with the electric switching mechanism to control the three-way valve to selectively connect the machine-controlled branch with the respiratory line or connect the hand-controlled branch with the respiratory line.

The three-way valve is applied in the anaesthesia machine. When the anaesthesia machine needs to perform leakage self-inspection, the anaesthesia machine can control the electric switching mechanism to input a drive force through a system program, thus controlling the connection state of the three-way valve. Specifically, in the leakage self-inspection under the machine-controlled mode, the machine-controlled branch is connected with the respiratory line, while in the leakage self-inspection under hand-controlled mode, the hand-controlled branch is connected with the respiratory line. After the anaesthesia machine has completed the leakage self-inspection under the machine-controlled mode, the anaesthesia machine automatically switches the connection state of the three-way valve through the system program configuration, so that the anaesthesia machine can automatically complete the leakage self-inspection under the hand-controlled mode. It can be seen that the full-automatic leakage self-inspection of the anaesthesia machine can be realized without manual intervention of the three-way valve during the leakage self-inspection of the anaesthesia machine.

### BRIEF DESCRIPTION OF THE DRAWINGS

FIG. 1 is a matching diagram of a three-way valve body and a valve core assembly according to an embodiment of this disclosure.
FIG. 2 is an exploded view of the structure shown in FIG. 1.
FIG. 3 is a sectional view of the structure shown in FIG. **1****,** wherein the valve core assembly is in a first working position.
FIG. 4 is a schematic diagram of the valve core assembly in the structure shown in FIG. 3, wherein the valve core assembly is in a second working position.
FIG. 5 is a sectional view of the structure shown in FIG. 1 along another sectional position, wherein the valve core assembly is in the first working position.
FIG. 6 is a schematic diagram of the valve core assembly in the structure shown in FIG. 5, wherein the valve core assembly is at the second working position.
FIG. 7 is a structural diagram of an electric switching mechanism according to an embodiment of this disclosure.
FIG. 8 is a sectional view of the structure shown in FIG. 7.
FIG. 9 is a sectional view of the structure shown in FIG. 7.
FIG. 10 is a schematic diagram of the structure shown in FIG. 9 in another state.
FIG. 11 is a structural diagram of a three-way valve according to an embodiment of this disclosure.
FIG. 12 is a principle schematic diagram of a three-way valve according to an embodiment of this disclosure.
FIG. 13 is a schematic diagram of a three-way valve according to another embodiment of this disclosure.
FIG. 14 is a schematic diagram of a gas path of an anaesthesia machine according to an embodiment of this disclosure.

Description of reference numbers:
Three-way valve body 11; Sliding cavity 110; First annular sealing surface 110a; Second annular sealing surface 110b; First channel 111; Second channel 112; Third channel 113; Stopper 112a; Installation space 114; Valve core assembly 12; Valve rod 121; Flange structure 1211; Step surface 121a; First segment 1212; Second segment 1213; Sealing portion 122; First guide portion 131; First sliding groove 131a; Second guide portion 132; Second sliding groove 132a; Elastic element 14; Toggle assembly 15; Toggle rod 151; Toggle sheet 152; Position detection device 16; First travel switch 161; Second travel switch 162; Electric switching mechanism 2; power portion 2'; Moving element 2"; Cylinder drive element 21; Valve base 210; Valve cavity 2101; Cavity with rod 2101a; Cavity without rod 2101b; Piston 211; Piston rod 212; Reset spring 213; Pilot solenoid valve 22; First connection rod 31; Slide block base 32; Second connection rod 33; Three-way valve 100; Respiratory line 200; Machine-controlled branch 300; Controller 400; Hand-controlled branch 500; Gas storage bag 501; Pressure discharge valve 502.

### DESCRIPTION OF THE PREFERRED EMBODIMENTS

It should be noted that, in the absence of conflict, the embodiments and the technical features in the embodiments of this disclosure can be combined with each other. The detailed description in the specific embodiments should be understood as an explanation of the purpose of the application and should not be regarded as an improper restriction on the application.

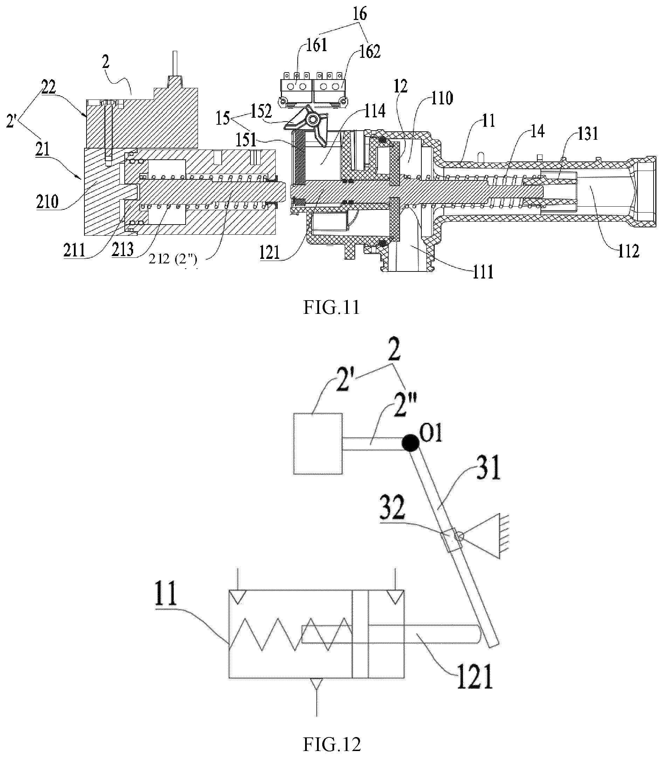

In the description of the embodiments of this disclosure, "left", "right", orientation or position relationship are based on the orientation or position relationship shown in FIG. 11. It should be understood that these orientation terms are only for the description convenience of this disclosure and simplifying the description, rather than indicating or implying that the device or element referred to must have a specific orientation, be constructed and operated in a specific orientation. Therefore, they cannot be understood as restrictions on this disclosure.

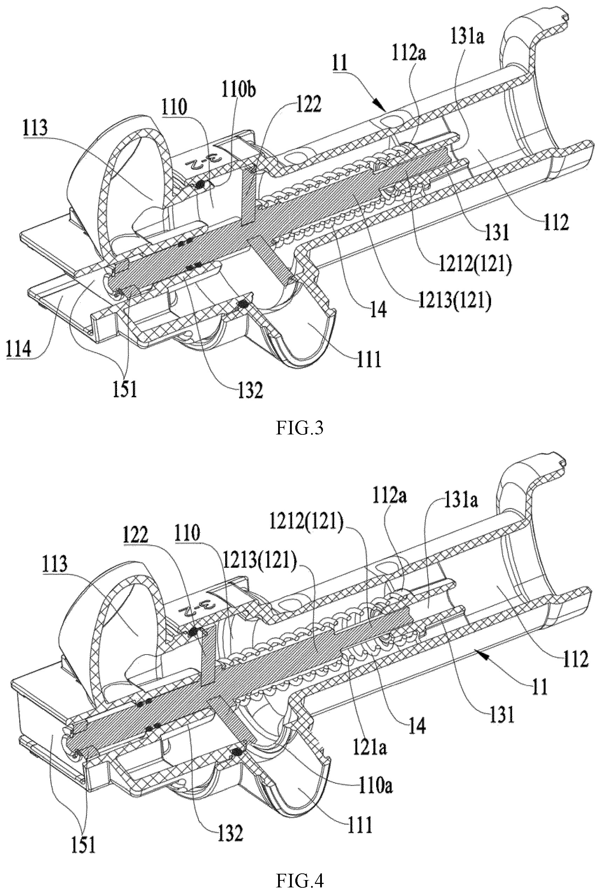

The embodiment of this disclosure has provided a three-way valve, which includes a three-way valve body 11, an electric switching mechanism 2 and a valve core assembly 12. Referring FIG. 3, the three-way valve body 11 has a sliding cavity 110, a first channel 111, a second channel 112 and a third channel 113 which are respectively connected with the sliding cavity 110. That is, the first channel 111, the second channel 112 and the third channel 113 are independently connected with the sliding cavity 110.

The valve core assembly 12 is arranged inside the three-way valve body 11. The valve core assembly 12 includes a valve rod 121 and a sealing portion 122 which is arranged at the valve rod 121. The sealing portion 122 is arranged inside the sliding cavity 110. The valve rod 121 can drive the sealing portion 122 to slide inside the sliding cavity 110 in a reciprocating manner.

The valve core assembly 12 further includes a first working position and a second working position. Referring FIG. 3, when the valve core assembly 12 is at the first working position, the sealing portion 122 closes the second channel 112 and the first channel 111 is connected with the third channel 113. Referring FIG. 4, when the valve core assembly 12 is at the second working position, the sealing portion 122 closes the third channel 113 and the first channel 111 is connected with the second channel 112.

The electric switching mechanism 2 is at least partially arranged outside of the three-way valve body 11, and can drive the valve rod 121 to move for driving the sealing portion 122 to slide inside the sliding cavity 110. It should be noted that the electric switching mechanism 2 in the embodiment of this disclosure outputs a drive force to drive the valve rod 121 to move after receiving an electric control signal, and the valve rod 121 then drives the sealing portion 122 to move, so as to realize the working position switching of the valve core assembly 12.

The application field of the three-way valve in the embodiment of this disclosure is not limited. The embodiment of this disclosure applies the three-way valve in the anaesthesia machine.

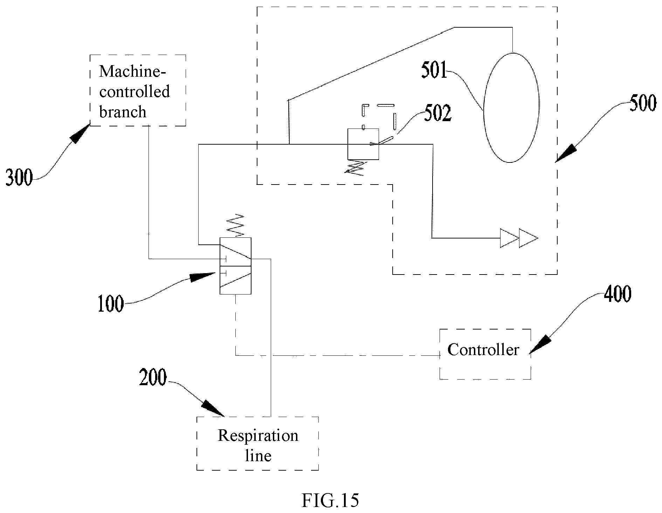

Referring FIG. 15, the anaesthesia machine includes a controller 400, a respiratory line 200, a hand-controlled branch 500, a machine-controlled branch 300 and the three-way valve 100 according to an embodiment of this disclosure. The first channel 111 is connected with the respiratory line 200, the second channel 112 is connected with the machine-controlled branch 300, the third channel 113 is connected with the hand-controlled branch 500, and the controller 400 is electrically connected with the electric switching mechanism 2 to control the three-way valve 100 to selectively connect the machine-controlled branch 300 with the respiratory line 200 or connect the hand-controlled branch 500 with the respiratory line 200.

It should be noted that the hand-controlled branch 500 is only effective during manual respiration. Specifically, the hand-controlled branch 500 is provided with an gas storage bag 501 and a pressure discharge valve 502. When the gas storage bag 501 is manually pinched too heavy, the gas storage bag 501 can discharge excess gas to prevent airway pressure injury.

When the anaesthesia machine needs to perform leak self-inspection or switch the ventilation mode for operation, or under other situation which requires checking, the anaesthesia machine can control the electric switching mechanism 2 to input the drive force through the system program, and then control the connection state of the three-way valve 100. In the embodiment of the application, the leakage self-inspection of the anaesthesia machine is described as an example.

For leakage self-inspection under the machine-controlled mode, the machine-controlled branch 300 is connected with the respiratory line 200. For leakage self-inspection under the hand-controlled mode, the hand-controlled branch 500 is connected with the respiratory line 200. After the anesthesia machine has completed the leakage self-inspection under the machine-controlled mode, the anaesthesia machine automatically switches the connection state of the three-way valve 100 through the system program configuration, so that the anaesthesia machine can automatically complete the leakage self-inspection under the hand-controlled mode. It can be seen that during the leakage self-inspection of the anaesthesia machine, there is no need to manually intervene the three-way valve 100, and the full-automatic leakage self-inspection of the anaesthesia machine can be realized.

Valve rod 121 has a certain length to facilitate sliding in the three-way valve body 11. In order to facilitate the drive connection between the valve rod 121 and the electric switching mechanism 2, the valve rod 121 extends to an outer surface of the three-way valve body 11 or extends outside of the three-way valve body 11, or in an embodiment not being part of the present invention, some structure of the electric switching mechanism 2 extends into the three-way valve body 11. It should be noted that no matter the valve rod 121 extends into the outer surface of the three-way valve body 11 or extends outside of the three-way valve body 11, or some structure of the electric switching mechanism 2 extends into the three-way valve body 11, it is necessary to ensure the sealing performance of the three-way valve body 11. That is, it is necessary to ensure that the three-way valve body 11 does not leak gas, so that the gas can only flow between the first channel 111 and the second channel 112, or between the first channel 111 and the third channel 113.

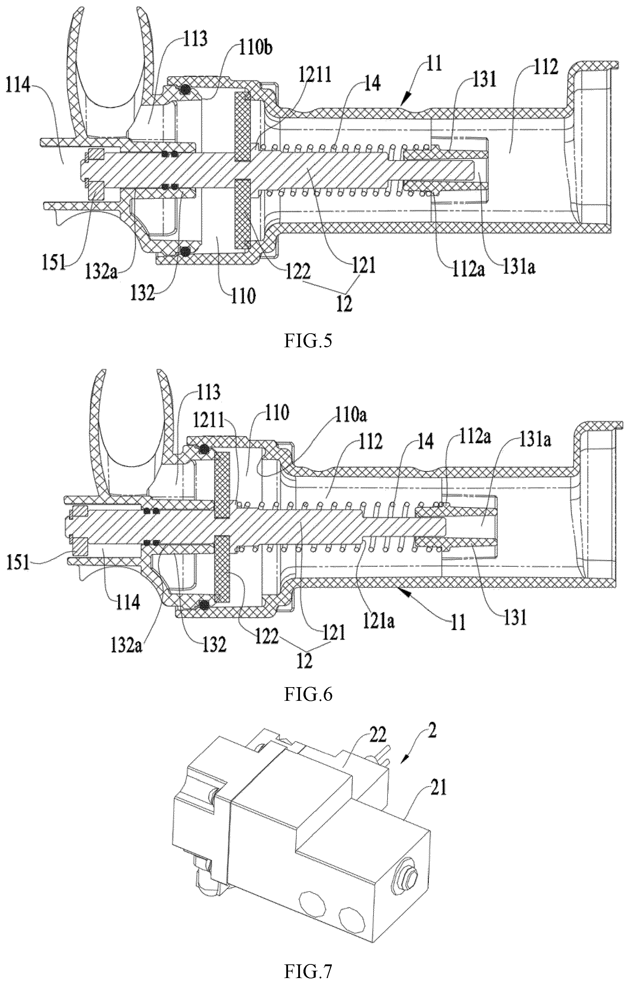

In an embodiment, referring to FIGs.5-6, the second channel 112 approximately extends along a straight line, the sliding cavity 110 approximately extends along a straight line, wherein the sliding cavity 110 and the second channel 112 are located at the same straight line, the second channel 112 is connected to one end of the sliding cavity 110 along the length direction, the third channel 113 is connected to the other end of the sliding cavity 110 along the length direction, and the connection position between the first channel 111 and the sliding cavity 110 is located at a side wall of the sliding cavity 110.

The specific structure and shape of the sealing portion 122 are not limited as long as the second channel 112 or the third channel 113 can be selectively closed. In an embodiment, the sealing portion 122 is a valve sheet, and the valve sheet is in a disc shape.

In an embodiment, referring to FIG. 4 and FIG. 6, a junction of the sliding cavity 110 and the second channel 112 is provided with a first annular sealing surface 110a, and the valve sheet abuts against the first annular sealing surface 110a hermetically to close the second channel 112. Specifically, an edge portion of the valve sheet along an axial side of the valve rod 121, abuts against the first annular sealing surface 110a, such that the first annular sealing surface 110a can fit the valve sheet well to ensure the gas tightness of the three-way valve and prevent the gas leakage. It should be noted that an extension dimension of the first annular sealing surface 110a along the radial direction is relatively small, and the first annular sealing surface 110a never contacts a surface of the valve rod 121, so as to prevent the first annular sealing surface 110a from blocking the gas flow.

In an embodiment, referring to FIG. 3 and FIG. 5, a junction of the sliding cavity 110 and the third channel 113 has a second annular sealing surface 110b, and the valve sheet abuts against the second annular sealing surface 110b hermetically to close the third channel 113. Specifically, an edge portion of the valve sheet along an axial opposite side of the valve rod 121, abuts against the second annular sealing surface 110b, such that the second annular sealing surface 110b can fit the valve sheet well to ensure the gas tightness of the three-way valve and prevent the gas leakage. It should be noted that, an extension dimension of the second annular sealing surface 110b along the radial direction is relatively small, and the second annular sealing surface 110b never contacts the surface of the valve rod 121, so as to prevent the second annular sealing surface 110b from blocking the gas flow.

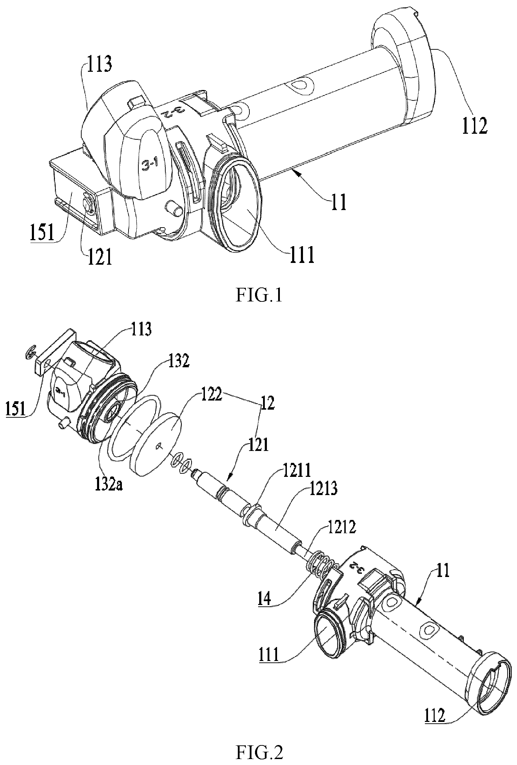

To facilitate the arrangement of the valve rod 121, refer to FIG. 1. The third channel 113 is bent and extended, and the second end of the valve rod 121 extends into the second channel 112. Referring to FIG. 5 and FIG. 6, the first end of the valve rod 121, which is opposite to the second channel, extends outside of the three-way valve body 11. That is, the third channel 113 is not coaxial with the valve rod 121 to avoid interfering the movement of the valve rod 121.

In an embodiment not being part of the invention, the above electric switching mechanism 2 drives the valve rod 121 to reciprocate, that is, the electric switching mechanism 2 can drive the valve core assembly 12 to switch from the first working position to the second working position, and can also drive the valve core assembly 12 to switch from the second working position to the first working position.

In another embodiment, please refer to FIG. 5 and FIG. 6. The above electric switching mechanism 2 only drives the valve rod 121 to slide in one direction. For example, the electric switching mechanism 2 only drive the valve rod 121 to slide from the left to the right of FIG. 5. That is, the drive valve core assembly 12 is switched from the second working position shown in FIG. 6 to the first working position shown in FIG. 5, and the valve core assembly 12 is driven to reset by other elastic structures. For example, the valve core assembly 12 is driven by an elastic structure to be switched from the first working position shown in FIG. 5 to the second working position shown in FIG. 6.

In order to guide the movement of the valve rod 121, in an embodiment referring to FIG. 3, the three-way valve includes a first guide portion 131 which is arranged inside the second channel 112. Refer to FIG. 5. The first guide portion 131 has a first sliding groove 131a which extends along a length direction of the valve rod 121, and the second end of the valve rod 121 penetrates into and is slidably arranged inside the first sliding groove 131a. The first sliding groove 131a plays a good role in guiding and positioning the movement of the valve rod 121.

In order to facilitate an axial positioning of the valve rod 121, in an embodiment referring to FIG. 3, the second end of the valve rod 121 includes a second segment 1213 and a first segment 1212 which are adjacent to each other. An outer diameter of the first segment 1212 is smaller than an outer diameter of the second segment 1213, such that, a step surface 121a is formed at a junction of the second segment 1213 and the first segment 1212. The first segment 1212 penetrates into and is slidably arranged inside the first sliding groove 131a, and a side of the first guide portion 131, which faces the sealing portion 122, abuts against the step surface 121a. Specifically, when the valve rod 121 moves from the position shown in FIG. 6 to the right of FIG. 6, until the step surface 121a abuts against the first guide portion 131, the first guide portion 131 acts as a barrier to prevent the valve rod 121 from continuing moving.

It should be noted that when the valve core assembly is in the first working position, the first guide portion 131 may or may not abut against the step surface 121a. Specifically, in an embodiment in which the first guide portion 131 never abuts against the step surface 121a, the first guide portion 131 plays a role of preventing overshooting of the valve rod 121.

In an embodiment, please refer to FIG. 3 and FIG. 5. The three-way valve includes a second guide portion 132 which is arranged inside the three-way valve body 11, the second guide portion 132 has a second sliding groove 132a which extends along the length direction of the valve rod 121. The second sliding groove 132a extends outside of the three-way valve body 11, and the first end of the valve rod 121 penetrates into and is slidably and hermetically arranged inside the second sliding groove 132a.

It should be noted that the first end of the valve rod 121 penetrates into and is slidably and hermetically arranged inside the second sliding groove 132a, means that the valve rod 121 can not only slide inside the second sliding groove 132a, but also prevent gas flow from leaking outside of the three-way valve body 11 along the second sliding groove 132a. Specifically, the first end of the valve rod 121 may be sleeved with a sealing ring, and the slidable and hermetical arrangement can be realized through the sealing ring.

It can be understood that in the embodiment in which the first guide portion 131 and the second guide portion 132 are provided at the same time, the first sliding groove 131a and the second sliding groove 132a are coaxially arranged so that the opposite ends of the valve rod 121 can penetrate into the first sliding groove 131a and the second sliding groove 132a at the same time.

Referring to FIG. 11, and according to the claimed invention, the electric switching structure only drives the valve rod 121 to slide in one direction. The three-way valve also includes an elastic element 14, which drives the valve core assembly 12 to reset.

In order to facilitate the positioning of the elastic element 14, in an embodiment, please refer to FIG. 4 and FIG. 6. The second channel 112 has a stopper 112a, and a first end of the elastic element 14 abuts against the stopper 112a, that is, the stopper 112a provides a reactive force support for the elastic element 14. A second end of the elastic element 14 can abut any appropriate position of the valve core assembly 12 to drive the valve core assembly 12 to move. For example, in one embodiment, the second end of the elastic element 14 abuts against the sealing portion 122, that is, the elastic element 14 directly applies a force to the sealing portion 122. For another example, in another embodiment, referring to FIG. 6, a flange structure 1211 is formed at the valve rod 121, and the second end of the elastic element 14 abuts against the flange structure 1211. The flange structure 1211 increases the contact area between the valve rod 121 and the elastic element 14, enabling a stable and reliable contact between the elastic element 14 and the valve rod 121.

There is no limit to the type of elastic element 14, which can be a spring, a rubber band, an elastic pad, etc. In the embodiment of this disclosure, the elastic element 14 is a spring, specifically, the spring is sleeved at the valve rod 121, the first end of the spring abuts against the stopper 112a, and the other end of the spring abuts against the flange structure 1211. The spring has large elastic deformation which satisfies the sliding stroke requirement of the sealing portion 122 and has reliable deformation recovery ability.

In an embodiment, referring to FIG. 11, the three-way valve also includes a position detection device 16 for detecting the working position of the valve core assembly 12. When the valve core assembly 12 is at the first working position, the position detection device 16 can trigger and generate a first position signal; and/or when the valve core assembly 12 is at the second working position, the position detection device 16 can trigger and generate a second position signal. Specifically, the position detection device 16 can be electrically connected with the controller 400 or other displayers to feed back the current working position of the valve core assembly 12. The feedback of the working position of the valve core assembly 12 through the position detection device 16 is convenient for the user or the controller to detect whether the valve core assembly 12 is switched in place, to determine whether the valve core assembly 12 is stuck or not, and to improve the reliability of the three-way valve.

In an embodiment, the above position detection device 16 triggers and generates the first position signal only when the valve core assembly 12 is at the first working position, and never triggers and generates signal when the valve core assembly 12 is at other positions. At this time, the default is that the valve core assembly 12 is at the second working position. In another embodiment, the above position detection device 16 triggers and generates the second position signal only when the valve core assembly 12 is at the second working position, and never triggers and generates signal when the valve core assembly 12 is at other positions. At this time, the default is that the valve core assembly 12 is at the first working position. In another embodiment, the above position detection device 16 triggers and generates the first position signal when the valve core assembly 12 is at the first working position, and triggers and generates the second position signal when the valve core assembly 12 is at the second working position. In this embodiment, whether the valve core assembly 12 is switched in place can be determined every time through the first position signal and the second position signal, which can further increase the working reliability of the three-way valve.

The specific type of the position detection device 16 is unlimited, as long as it can detect the corresponding position of the valve core assembly 12. For example, the position detection device 16 may be a Hall sensor or a photoelectric sensor, and there is no limitation here.

It should be noted that since the valve rod 121 and the sealing portion 122 move synchronously, the position detection device 16 can detect any portion of the valve rod 121, any portion of the sealing portion 122, and other structures which are fixedly connected with the valve core assembly 12.

In an embodiment, referring to FIG. 11, the position detection device 16 includes a first travel switch 161 and a second travel switch 162, the three-way valve includes a toggle assembly 15, and the valve rod 121 is in drive connection with the toggle assembly 15 to selectively drive the toggle assembly 15 to trigger the first travel switch 161 or the second travel switch 162. When the valve rod 121 moves, it drives the toggle assembly 15 to move, thus trigging the first travel switch 161 or the second travel switch 162 by the toggle assembly 15. For example, when the valve rod 121 moves to the first working position, the toggle assembly 15 contacts the first travel switch 161 to trigger the first travel switch 161. When the valve rod 121 moves to the second working position, the toggle assembly 15 contacts the second travel switch 162 to trigger the second travel switch 162. In this way, the position information can be fed back at both working positions of the valve rod 121.

The specific structure of the toggle assembly 15 is unlimited, as long as it is convenient to toggle the first travel switch 161 and the second travel switch 162. In an embodiment, please continue to refer to FIG. 11. The toggle assembly 15 includes a toggle rod 151 and a toggle sheet 152, wherein the toggle sheet 152 rotates relative to the three-way valve body 11. The toggle sheet 152 can be rotatably arranged on the three-way valve body 11 or on other structures as long as it can rotate.

The toggle rod 151 is fixedly connected with the first end of the valve rod 121. The toggle rod 151 can drive the toggle sheet 152 to rotate forward or backward during the linear reciprocation movement following the valve rod 121, so as to selectively trigger the first travel switch 161 or the second travel switch 162. For example, when the valve rod 121 moves to a certain position toward the left of FIG. 11, the valve rod 121 contacts the toggle sheet 152 and drives the toggle sheet 152 to rotate forward along the clockwise direction in FIG. 11, the toggle sheet 152 contacts the second travel switch 162 and triggers the second travel switch 162. When the valve rod 121 moves to a certain position toward the right of FIG. 11, the valve rod 121 contacts with the toggle sheet 152 and drives the toggle sheet 152 to rotate backward along the counterclockwise direction in FIG. 11, the toggle sheet 152 contacts with the first travel switch 161 and triggers the first travel switch 161.

It should be noted that the forward rotation and backward rotation are relative to each other, and never mean that a specific direction is the forward rotation or the backward rotation. For example, the clockwise rotation of the toggle sheet 152 in FIG. 11 can be defined as the forward rotation, and the counterclockwise rotation of the toggle sheet 152 in FIG. 11 can be defined as the backward rotation. Alternatively, the clockwise rotation of the toggle sheet 152 in FIG. 11 can be defined as the backward rotation, and the counterclockwise rotation of the toggle sheet 152 in FIG. 11 can be defined as the forward rotation.

In an embodiment, please refer to FIG. 3 and FIG. 11 in combination. An installation space 114 is formed outside of the three-way valve body 11, and the toggle rod 151 is arranged inside the installation space 114. Specifically, the toggle rod 151 is approximately perpendicular to the valve rod 121, the first end of the toggle rod 151 is fixedly connected with the valve rod 121, and the second end of the toggle rod 151 is configured to toggle the toggle sheet 152. The installation space 114 plays a guiding role for the movement of the toggle rod 151. The toggle sheet 152 is rotatably connected with other structures, that is, the toggle sheet 152 is not connected with the three-way valve body 11, and at least part of the structure of the toggle sheet 152 and the position detection device 16 are located outside the installation space 114. In this way, the toggle sheet 152 and the position detection device 16 can avoid occupying the installation space 114, thus the three-way valve body 11 can be made compact.

According to the claimed invention, the electric switching structure includes a power portion 2' and a moving element 2" which are in drive connection. The power portion 2' provides power for the moving element 2", specifically, the power portion 2' drives the moving element 2" to slide in a reciprocating manner or rotate, and the electric switching mechanism 2 drives the valve rod 121 to move via the moving element 2".

In an embodiment not being part of the invention, the moving element 2" drives the valve rod 121 to reciprocate, that is, specifically, the moving element 2" can drive the valve core assembly 12 to switch from the first working position to the second working position, and can also drive the valve core assembly 12 to switch from the second working position to the first working position.

The specific form of the moving element 2" is unlimited. When the moving element 2" is in a rod shape, the moving element 2" and the valve rod 121 can be arranged along the same straight line or can be staggered. There is no restriction for this here.

The moving element 2" can be directly connected with the valve rod 121, or indirectly connected with the valve rod 121 through other structures, so that the valve rod 121 and the moving element 2" move synchronously.

Referring to FIG. 11, the moving element 2" and the valve rod 121 are selectively separated or in drive connection to push the valve rod 121 to slide in one direction, for example, to push the valve core assembly 12 to switch from the second working position to the first working position. According to the claimed invention, in order to ensure the reset of the valve core assembly 12, the three-way valve includes the above elastic element 14, which is in drive connection with the valve core assembly 12 to drive the valve core assembly 12 to reset, such as to drive the valve core assembly 12 to switch from the first working position to the second working position. That is, in this embodiment, the moving element 2" and the elastic element 14 exert forces in the opposite directions at the valve core assembly 12 to jointly drive the valve core assembly 12 to switch between the first working position and the second working position. In this embodiment, the power portion 2' can drive the moving element 2" to reciprocate or can drive the moving element 2" to rotate.

The moving element 2" directly and separably abuts against the end of the valve rod 121, that is, the moving element 2" directly pushes the valve rod 121 to slide in one direction. When the moving element 2" is in a rod shape, the moving element 2" and the valve rod 121 can be arranged along the same straight line or can be staggered. There is no restriction for this here.

The moving element 2" can also indirectly push the valve rod 121 to slide in one direction through other structures.

Specifically, in an embodiment, please refer to FIG. 12, the power portion 2' drives the moving element 2" to reciprocate. Specifically, the three-way valve includes a first connection rod 31 and a slide block base 32. The first connection rod 31 penetrates into and is slidably arranged inside the slide block base 32. An end of the moving element 2" is hinged with a first end of the first connection rod 31, the hinged point is point O1 in FIG. 12, one end of the valve rod 121 slidably contacts with a second end of the first connection rod 31. The moving element 2" and the valve rod 121 are located at the same side of the first connection rod 31, and the slide block base 32 is hinged with other installation structure. That is, the moving element 2, the first connection rod 31 and the slide block base 32 form a crank sliding mechanism. When the moving element 2" extends along a direction towards the first connection rod 31, the moving element 2" drives the first connection rod 31 to swing in a clockwise direction in the FIG. 12 with the slide block base 32 as the center. At the same time, the first connection rod 31 also generates a certain linear displacement inside the slide block base 32, so that the moving element 2" can always maintain a linear movement and avoid the clamping stagnation of the moving element 2".

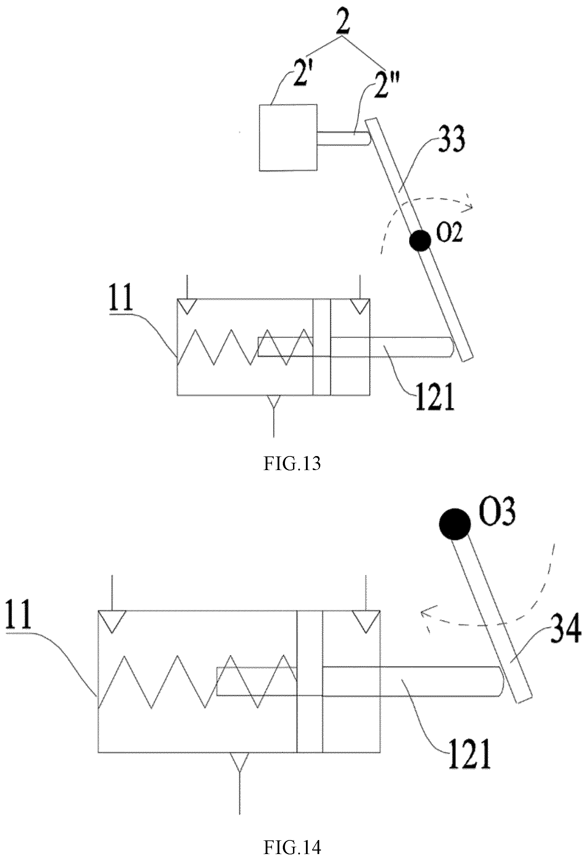

In another embodiment, referring to FIG. 13, the power portion 2' drives the moving element 2" to reciprocate. Specifically, the three-way valve includes a second connection rod 33, and an end of the moving element 2" slidably contacts with a first end of the second connection rod 33, the valve rod 121 slidably contacts with a second end of the second connection rod 33, the moving element 2" and the valve rod 121 are located at the same side of the second connection rod 33, and a roughly middle position of the second connection rod 33 is rotatably connected with other installation structure, so that the second connection rod 33 has a rotation center O2. The moving element 2" extends towards the second connection rod 33, drives the second connection rod 33 to rotate around the rotation center O2 along a direction of a dotted arrow in FIG. 13. The second end of the second connection rod 33 forces the valve rod 121 to retract towards the inside of the three-way valve body, that is, the valve core assembly 12 is switched from the first working position to the second working position. When the valve core assembly 12 needs to be switched from the second working position to the first working position, the moving element 2" moves away from the second connection rod 33 to eliminate the drive of the moving element 2" at the valve rod 121, and the valve rod 121 is reset under the action of the above elastic element, that is, the valve core assembly 12 is switched from the second working position to the first working position.

In a further embodiment, referring to FIG. 14, the power portion 2' drives the moving element 2" to rotate. Specifically, the three-way valve includes a third connection rod 34, and the moving element 2" is rotatably connected with the third connection rod 34. The rotation connection point is O3, and the moving element 2" drives the third connection rod 34 to rotate around the rotation connection point O3. It should be noted that the moving element 2" and the power portion 2' are not shown in FIG. 14. In the state shown in FIG. 14, the third connection rod 34 rotates along a direction of a dotted arrow with the rotation connection point O3 as a center to drive the three-way valve to switch. In this embodiment, the power portion may be a rotation motor.

The specific structural form of the above power portion 2' is unlimited, as long as it can provide power for the moving part 2".

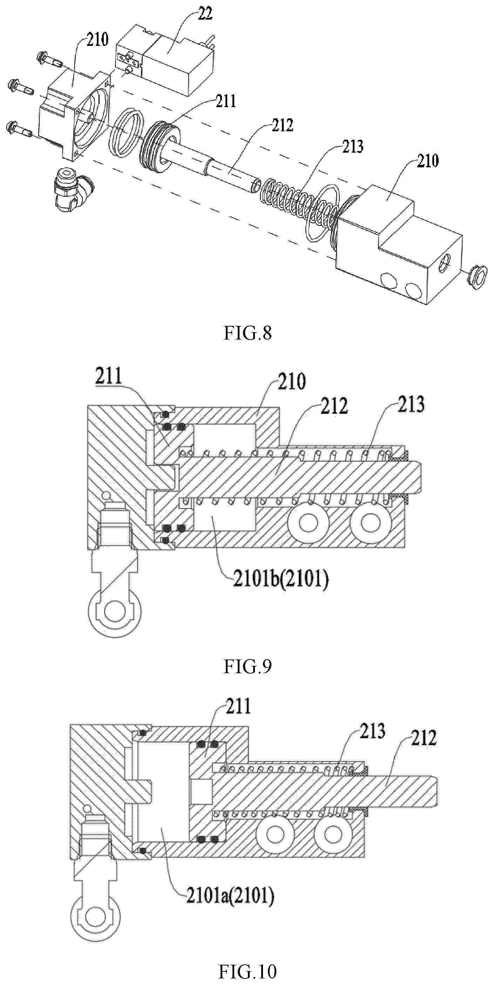

According to the invention, and referring to FIGs.7 and 8, the power portion 2' includes a cylinder drive element 21 and a pilot solenoid valve 22, and the moving element 2" is a piston rod 212. Specifically, the cylinder drive element 21 includes a valve base 210 and a piston 211 which is fixedly connected with the piston rod 212. Referring to FIGs. 9 and 10, the valve base 210 has a valve cavity 2101 and a first gas inlet connected with the valve cavity 2101. The piston 211 separates the valve cavity 2101 into a cavity with rod 2101a and a cavity without rod 2101b. The first gas inlet is connected with the cavity without rod 2101b, and the pilot solenoid valve 22 can connect the first gas inlet with the gas source, so that the piston rod 212 drives the valve rod 121 to move. It should be noted that the specific type and structure of the pilot solenoid valve 22 are not limited. For example, in an embodiment, the valve core opening of the pilot solenoid valve 22 can be adjusted. For example, during specific implementation, valve core opening of the pilot solenoid valve 22 can be adjusted by controlling current or voltage applied to a control terminal of the pilot solenoid valve 22, so as to control the gas flows which pass through the solenoid pilot valve 22, thus controlling a switching speed of the three-way valve. In another embodiment, gas intaking speed or gas discharging speed of the gas path can be controlled by arranging a throttle valve or orifice on the gas path to increase the gas resistance, so that the switching speed of the three-way valve can be controlled. It is understandable that the size of the throttle valve can be fixed or adjustable, and the orifice diameter can be fixed or adjustable.

Similarly, in this embodiment, the piston rod 212 can drive the valve rod 121 to reciprocate or slide in one direction.

When the piston rod 212 needs to extend out from the valve cavity 2101, the pilot solenoid valve 22 is powered on, and the gas from the gas source enters the cavity without rod 2101b from the first gas inlet through the pilot solenoid valve 22. The gas pushes the piston 211 to move towards the cavity without rod 2101a, thereby extending out the piston rod 212 to drive the valve rod 121 to move.

When the piston rod 212 needs to reset, the cavity with rod 2101a can be connected with the gas source to drive the piston 211 to move towards the cavity without rod 2101b, thus driving the piston rod 212 to reset. Therefore, in this embodiment, the valve base 210 also has a second gas inlet which is connected with the cavity without rod 2101b, and the pilot solenoid valve 22 can selectively connect the gas source with the first gas inlet or the second gas inlet. Specifically, when the piston rod 212 needs to extend out, the gas, which enters from the first gas inlet and then is discharged from the second gas inlet, pushes the piston 211 to move towards the cavity with rod 2101a, so that the piston rod 212 extends out. When the piston rod 212 needs to retract, the gas, which enters from the second gas inlet and then is discharged from the first gas inlet, pushes the piston 211 to move towards the cavity without rod 2101b, so that the piston rod 212 retracts.

In another embodiment, please continue to refer to FIGS. 9 and 10. The cavity with rod 2101a is connected with atmosphere, that is, the cavity with rod 2101a is not closed, and the cylinder drive element 21 includes a return spring 213, which is sleeved at the piston rod 212 and can drive the piston rod 212 to reset. In this embodiment, the piston rod 212 is driven to reset by the return spring 213, and there is no need to seal the cavity with rod 2101a or control the gas intake and gas discharge of the cavity with rod 2101a through the pilot solenoid valve 22, which can reduce the manufacture difficulty of the cylinder drive element 21 and simplify the gas path control. It should be noted that in this embodiment, during the reset process of the piston rod 212, the gas in the cavity without rod 2101b needs to be discharged and the gas in the cavity without rod 2101b can be discharged through the pilot solenoid valve 22 mentioned above, or through other valves, which are not limited here.

In another embodiment, the electric switching mechanism 2 is a linear motor, which includes a stator and a mover. The mover can reciprocate relative to the stator. Specifically, the power portion 2' is the stator of the linear motor, and the moving element 2" is the mover of the linear motor.

It should be noted that in the embodiment shown in FIG. 12, the moving element 2" can be the above piston rod or the above mover. Similarly, in the embodiment shown in FIG. 13, the moving element 2" can be the above piston rod or the above mover.

The embodiments/implementations provided in this disclosure can be combined with each other without contradiction.

The above descriptions are only preferred embodiments of this disclosure and are not intended to limit this disclosure. For those skilled in the art, this disclosure may have various changes and modifications. The scope of protection of the invention shall be subject to the scope of protection of the claims.

## Claims

1. A three-way valve (100), comprising:
a three-way valve body (11), wherein the three-way valve body (11) is provided with a sliding cavity (110), and a first channel (111), a second channel (112) and a third channel (113) which are respectively connected with the sliding cavity (110);
a valve core assembly (12) which is arranged inside the three-way valve body (11), wherein the valve core assembly (12) comprises a valve rod (121) and a sealing portion (122) which is arranged at the valve rod (121), the valve rod (121) is configured to drive the sealing portion (122) to slide inside the sliding cavity (110) in a reciprocating manner; wherein the valve core assembly (12) further comprises a first working position and a second working position, when the valve core assembly (12) is at the first working position, the sealing portion (122) closes the second channel (112), and the first channel (111) is connected with the third channel (113), when the valve core assembly (12) is at the second working position, the sealing portion (122) closes the third channel (113), and the first channel (111) is connected with the second channel (112); and
an electric switching mechanism (2) which is at least partially arranged outside of the three-way valve body (11) and comprises a power portion (2') and a moving element (2") which are in drive connection, wherein the electric switching mechanism (2) is configured to drive the valve rod (121) to move for driving the sealing portion (122) to slide inside the sliding cavity (110);
wherein, the valve rod (121) is able to extend to an outer surface of the three-way valve body (11) or extend outside of the three-way valve body (11), and the moving element (2") of the electric switching mechanism (2) and the valve rod (121) are selectively separated or in drive connection, to push the valve rod (121) to slide in one direction, wherein the three-way valve (100) further comprises an elastic element (14) which is in drive connection with the valve core assembly (12) to exert a force in an opposite direction of the moving element (2"), to drive the valve core assembly (12) to reset; and
wherein the power portion (2') of the electric switching mechanism (2) comprises a cylinder drive element (21) and a pilot solenoid valve (22), the moving element (2") is a piston rod (212), the cylinder drive element (21) comprises a valve base (210) and a piston (211) which is fixedly connected with the piston rod (212), the valve base (210) is provided with a valve cavity (2101) and a first gas inlet which is connected with the valve cavity (2101), and wherein the pilot solenoid valve (22) is configured to connect the first gas inlet with a gas source, so that the piston rod (212) is able to drive the valve rod (121) to move.

2. The three-way valve (100) according to claim 1, wherein a second end of the valve rod (121) extends into the second channel (112), and the second channel (112) is provided with a stopper (112a) and a first end of the elastic element (14) abuts against the stopper (112a);
a second end of the elastic element (14) abuts against the sealing portion (122), or a flange structure (1211) is formed at the valve rod (121) and a second end of the elastic element (14) abuts against the flange structure (1211).

3. The three-way valve (100) according to claim 1, wherein the three-way valve (100) further comprises a first guide portion (131) which is arranged inside the second channel (112); wherein the first guide portion (131) is provided with a first sliding groove (131a) which extends along a length direction of the valve rod (121), and a second end of the valve rod (121) penetrates into and is slidably arranged inside the first sliding groove (131a).

4. The three-way valve (100) according to claim 3, wherein the second end of the valve rod (121) comprises a first segment (1212) and a second segment (1213) which are adjacent to each other; wherein an outer diameter of the first segment (1212) is smaller than an outer diameter of the second segment (1213), such that a step surface (121a) is formed at a junction of the second segment (1213) and the first segment (1212); wherein the first segment (1212) penetrates into and is slidably arranged inside the first sliding groove (131a), and a side of the first guide portion (131), which faces the sealing portion (122), abuts against the step surface (121a).

5. The three-way valve (100) according to claim 1, wherein
the three-way valve (100) further comprises a second guide portion (132) which is arranged inside the three-way valve body (11); wherein the second guide portion (132) is provided with a second sliding groove (132a) which extends outside of the three-way valve body (11) along a length direction of the valve rod (121), wherein a first end of the valve rod (121) penetrates into and is slidably and hermetically arranged inside the second sliding groove (132a).

6. The three-way valve (100) according to claim 1, wherein the sealing portion (122) is a valve sheet, wherein a junction of the sliding cavity (110) and the second channel (112) is provided with a first annular sealing surface (110a), and the valve sheet is configured to hermetically abut against the first annular sealing surface (110a) to close the second channel (112); and/or a junction of the sliding cavity (110) and the third channel (113) is provided with a second annular sealing surface (110b), and the valve sheet is configured to hermetically abut against the second annular sealing surface (110b) to close the third channel (113).

7. The three-way valve (100) according to claim 6, wherein when the valve core assembly (12) is at the first working position, an edge portion of the valve sheet along an axial side of the valve rod (121) hermetically abuts against the first annular sealing surface (110a), and when the valve core assembly (12) is at the second working position, an edge portion of the valve sheet along an axial opposite side of the valve rod (121) hermetically abuts against the second annular sealing surface (110b).

8. The three-way valve (100) according to claim 1, wherein the three-way valve (100) further comprises a position detection device (16) which is configured to detect a working position of the valve core assembly (12); wherein when the valve core assembly (12) is at the first working position, the position detection device (16) is configured to trigger and generate a first position signal; and/or when the valve core assembly (12) is at the second working position, the position detection device (16) is configured to trigger and generate a second position signal.

9. The three-way valve (100) according to claim 8, wherein the position detection device (16) comprises a first travel switch (161) and a second travel switch (162), the three-way valve (100) comprises a toggle assembly (15), wherein the valve rod (121) is in drive connection with the toggle assembly (15) to selectively drive the toggle assembly (15) to trigger the first travel switch (161) or the second travel switch (162).

10. The three-way valve (100) according to claim 9, wherein the toggle assembly (15) comprises a toggle rod (151) and a toggle sheet (152), the toggle sheet (152) rotates relative to the three-way valve body (11), the toggle rod (151) is fixedly connected with a first end of the valve rod (121); wherein the toggle rod (151) is configured to drive the toggle sheet (152) to rotate forward or backward during a linear reciprocation movement following the valve rod (121), so as to selectively trigger the first travel switch (161) or the second travel switch (162); preferably, an installation space (114) is formed outside of the three-way valve body (11), and the toggle rod (151) is arranged inside the installation space (114).

11. The three-way valve (100) according to claim 1, wherein the power portion (2') drives the moving element (2") to slide in a reciprocating manner or rotate, and the electric switching mechanism (2) drives the valve rod (121) to slide through the moving element (2").

12. The three-way valve (100) according to claim 1, wherein the cavity with rod (2101a) is connected with atmosphere, the cylinder drive element (21) further comprises a return spring (213), which is sleeved at the piston rod (212) and is configured to drive the piston rod (212) to reset.

13. The three-way valve (100) according to claim 11, wherein the electric switching mechanism (2) is a linear motor, the power portion (2') is a stator of the linear motor, and the moving element (2") is a mover of the linear motor.

14. The three-way valve (100) according to claim 1, wherein the second channel (112) extends approximately along a straight line, the sliding cavity (110) extends approximately along a straight line, and the sliding cavity (110) and the second channel (112) are located at the same straight line.

15. An anaesthesia machine, comprising a controller (400), a respiratory line (200), a hand-controlled branch (500), a machine-controlled branch (300) and the three-way valve (100) according to any one of claims 1-14, wherein the first channel (111) is connected with the respiratory line (200), the second channel (112) is connected with the machine-controlled branch (300), the third channel (113) is connected with the hand-controlled branch (500), and the controller (400) is electrically connected with the electric switching mechanism (2) to control the three-way valve (100) to selectively connect the machine-controlled branch (300) with the respiratory line (200) or connect the hand-controlled branch (500) with the respiratory line (200).

## Patentansprüche

1. Ein Dreiwegeventil (100), das Folgendes umfasst:
einen Dreiwegeventilkörper (11), wobei der Dreiwegeventilkörper (11) mit einem Gleithohlraum (110) sowie einem ersten Kanal (111), einem zweiten Kanal (112) und einem dritten Kanal (113) versehen ist, die jeweils mit dem Gleithohlraum (110) verbunden sind;
eine Ventilkernbaugruppe (12), die innerhalb des Dreiwegeventilkörpers (11) angeordnet ist, wobei die Ventilkernbaugruppe (12) eine Ventilstange (121) und einen Dichtungsabschnitt (122) umfasst, der an der Ventilstange (121) angeordnet ist, die Ventilstange (121) so ausgebildet ist, dass sie den Dichtungsabschnitt (122) dazu antreibt, sich innerhalb des Gleithohlraums (110) hin- und herbewegend zu verschieben; die Ventilkernbaugruppe (12) ferner eine erste Arbeitsposition und eine zweite Arbeitsposition umfasst, wobei, wenn sich die Ventilkernbaugruppe (12) in der ersten Arbeitsposition befindet, der Dichtungsabschnitt (122) den zweiten Kanal (112) verschließt und der erste Kanal (111) mit dem dritten Kanal (113) verbunden ist; wenn sich die Ventilkernbaugruppe (12) in der zweiten Arbeitsposition befindet, der Dichtungsabschnitt (122) den dritten Kanal (113) verschließt und der erste Kanal (111) mit dem zweiten Kanal (112) verbunden ist; und
einen elektrischen Schaltmechanismus (2), der zumindest teilweise außerhalb des Dreiwegeventilkörpers (11) angeordnet ist und einen Antriebsteil (2') sowie ein bewegliches Element (2") umfasst, die in Antriebsverbindung stehen, wobei der elektrische Schaltmechanismus (2) so ausgebildet ist, dass er die Ventilstange (121) antreibt, um den Dichtungsabschnitt (122) zum Gleiten innerhalb des Gleithohlraums (110) zu bewegen;
wobei sich die Ventilstange (121) bis zu einer Außenfläche des Dreiwegeventilkörpers (11) erstrecken oder aus dem Dreiwegeventilkörper (11) herausragen kann und das bewegliche Element (2") des elektrischen Schaltmechanismus (2) und die Ventilstange (121) wahlweise voneinander getrennt sind oder in Antriebsverbindung stehen, um die Ventilstange (121) in eine Richtung zu verschieben,
wobei das Dreiwegeventil (100) ferner ein elastisches Element (14) umfasst, das in Antriebsverbindung mit der Ventilkernbaugruppe (12) steht, um eine Kraft in einer dem Bewegungselement (2") entgegengesetzten Richtung auszuüben, um die Ventilkernbaugruppe (12) in die Ausgangsstellung zurückzuführen; und
wobei der Antriebsteil (2') des elektrischen Schaltmechanismus (2) ein Zylinderantriebselement (21) und ein Vorsteuermagnetventil (22) umfasst, das bewegliche Element (2") eine Kolbenstange (212) ist, das Zylinderantriebselement (21) eine Ventilbasis (210) und einen Kolben (211) umfasst, der fest mit der Kolbenstange (212) verbunden ist, die Ventilbasis (210) mit einem Ventilhohlraum (2101) und einem ersten Gaseinlass versehen ist, der mit dem Ventilhohlraum (2101) verbunden ist, und das Vorsteuermagnetventil (22) so ausgebildet ist, dass es den ersten Gaseinlass mit einer Gasquelle verbindet, sodass die Kolbenstange (212) die Ventilstange (121) zu einer Bewegung antreiben kann.

2. Das Dreiwegeventil (100) nach Anspruch 1, wobei sich ein zweites Ende der Ventilstange (121) in den zweiten Kanal (112) erstreckt und der zweite Kanal (112) mit einem Anschlag (112a) versehen ist und ein erstes Ende des elastischen Elements (14) an dem Anschlag (112a) anliegt;
ein zweites Ende des elastischen Elements (14) an dem Dichtungsabschnitt (122) anliegt, oder an der Ventilstange (121) eine Flanschstruktur (1211) ausgebildet ist und ein zweites Ende des elastischen Elements (14) an der Flanschstruktur (1211) anliegt.

3. Das Dreiwegeventil (100) nach Anspruch 1, wobei das Dreiwegeventil (100) ferner einen ersten Führungsabschnitt (131) umfasst, der innerhalb des zweiten Kanals (112) angeordnet ist; wobei der erste Führungsabschnitt (131) mit einer ersten Gleitnut (131a) versehen ist, die sich entlang einer Längsrichtung der Ventilstange (121) erstreckt, und ein zweites Ende der Ventilstange (121) in die erste Gleitnut (131a) hineinragt und darin gleitend angeordnet ist.

4. Das Dreiwegeventil (100) nach Anspruch 3, wobei das zweite Ende der Ventilstange (121) ein erstes Segment (1212) und ein zweites Segment (1213) umfasst, die aneinander angrenzen; wobei der Außendurchmesser des ersten Segments (1212) kleiner ist als der Außendurchmesser des zweiten Segments (1213), so dass an der Verbindungsstelle des zweiten Segments (1213) und des ersten Segments (1212) eine Stufenfläche (121a) gebildet wird; wobei das erste Segment (1212) in die erste Gleitnut (131a) hineinragt und darin verschiebbar angeordnet ist und eine Seite des ersten Führungsabschnitts (131), die dem Dichtungsabschnitt (122) zugewandt ist, an der Stufenfläche (121a) anliegt.

5. Das Dreiwegeventil (100) nach Anspruch 1, wobei das Dreiwegeventil (100) ferner einen zweiten Führungsabschnitt (132) umfasst, der innerhalb des Dreiwegeventilkörpers (11) angeordnet ist; der zweite Führungsabschnitt (132) mit einer zweiten Gleitnut (132a) versehen ist, die sich außerhalb des Dreiwegeventilkörpers (11) entlang einer Längsrichtung der Ventilstange (121) erstreckt, wobei ein erstes Ende der Ventilstange (121) in die zweite Gleitnut (132a) hineinragt und darin gleitbar und dichtend angeordnet ist.

6. Das Dreiwegeventil (100) nach Anspruch 1, wobei der Dichtungsabschnitt (122) eine Ventilplatte ist, eine Verbindungsstelle des Gleithohlraums (110) und des zweiten Kanals (112) mit einer ersten ringförmigen Dichtfläche (110a) versehen ist und die Ventilplatte so ausgebildet ist, dass sie dichtend an der ersten ringförmigen Dichtfläche anliegt (110a), um den zweiten Kanal (112) zu verschließen; und/oder eine Verbindungsstelle des Gleithohlraums (110) und des dritten Kanals (113) mit einer zweiten ringförmigen Dichtfläche (110b) versehen ist und die Ventilplatte so ausgebildet ist, dass sie dichtend an der zweiten ringförmigen Dichtfläche (110b) anliegt, um den dritten Kanal (113) zu verschließen.

7. Das Dreiwegeventil (100) nach Anspruch 6, wobei, wenn sich die Ventilkernbaugruppe (12) in der ersten Arbeitsposition befindet, ein Randabschnitt der Ventilplatte entlang einer axialen Seite der Ventilstange (121) dichtend an der ersten ringförmigen Dichtfläche (110a) anliegt, und wenn sich die Ventilkernbaugruppe (12) in der zweiten Arbeitsposition befindet, ein Randabschnitt der Ventilplatte entlang einer axial gegenüberliegenden Seite der Ventilstange (121) dichtend an der zweiten ringförmigen Dichtfläche (110b) anliegt.

8. Das Dreiwegeventil (100) nach Anspruch 1, wobei das Dreiwegeventil (100) ferner eine Positionserfassungsvorrichtung (16) umfasst, die dazu ausgelegt ist, eine Arbeitsposition der Ventilkernbaugruppe (12) zu erfassen; wobei, wenn sich die Ventilkernbaugruppe (12) in der ersten Arbeitsposition befindet, die Positionserfassungsvorrichtung (16) dazu ausgelegt ist, ein erstes Positionssignal auszulösen und zu erzeugen; und/oder wenn sich die Ventilkernbaugruppe (12) in der zweiten Arbeitsposition befindet, die Positionserfassungsvorrichtung (16) so ausgelegt ist, dass sie ein zweites Positionssignal auslöst und erzeugt.

9. Das Dreiwegeventil (100) nach Anspruch 8, wobei die Positionserfassungsvorrichtung (16) einen ersten Wegschalter (161) und einen zweiten Wegschalter (162) umfasst, das Dreiwegeventil (100) eine Kniehebelbaugruppe (15) umfasst, wobei die Ventilstange (121) in Antriebsverbindung mit der Kniehebelbaugruppe (15) steht, um die Kniehebelbaugruppe (15) selektiv anzutreiben und so den ersten Wegschalter (161) oder den zweiten Wegschalter (162) auszulösen.

10. Das Dreiwegeventil (100) nach Anspruch 9, wobei die Kniehebelbaugruppe (15) eine Kniehebelstange (151) und eine Kniehebelplatte (152) umfasst, sich die Kniehebelplatte (152) relativ zum Dreiwegeventilkörper (11) dreht, die Kniehebelstange (151) fest mit einem ersten Ende der Ventilstange (121) fest verbunden ist; wobei die Kniehebelstange (151) so ausgebildet ist, dass sie die Kniehebelplatte (152) während einer linearen Hin- und Herbewegung, die der Ventilstange (121) folgt, dazu antreibt, sich vorwärts oder rückwärts zu drehen, um wahlweise den ersten Wegschalter (161) oder den zweiten Wegschalter (162) auszulösen; vorzugsweise ist ein Einbauraum (114) außerhalb des Dreiwegeventilkörpers (11) ausgebildet und die Kniehebelstange (151) innerhalb des Einbauraums (114) angeordnet.

11. Das Dreiwegeventil (100) nach Anspruch 1, wobei der Antriebsabschnitt (2') das bewegliche Element (2") dazu antreibt, sich hin- und hergehend zu verschieben oder zu drehen, und der elektrische Schaltmechanismus (2) die Ventilstange (121) antreibt, um durch das bewegliche Element (2") zu gleiten.

12. Das Dreiwegeventil (100) nach Anspruch 1, wobei der Hohlraum mit Stange (2101a) mit der Atmosphäre verbunden ist, das Zylinderantriebselement (21) ferner eine Rückstellfeder (213) umfasst, die auf die Kolbenstange (212) aufgeschoben und so ausgebildet ist, dass sie die Kolbenstange (212) in die Ausgangsstellung zurückführt.

13. Das Dreiwegeventil (100) nach Anspruch 11, wobei der elektrische Schaltmechanismus (2) ein Linearmotor ist, der Antriebsabschnitt (2') ein Stator des Linearmotors und das bewegliche Element (2") ein Antriebselement des Linearmotors.

14. Das Dreiwegeventil (100) nach Anspruch 1, wobei sich der zweite Kanal (112) annähernd entlang einer geraden Linie erstreckt, der Gleitkanal (110) annähernd entlang einer geraden Linie erstreckt und der Gleitkanal (110) sowie der zweite Kanal (112) auf derselben geraden Linie liegen.

15. Ein Anästhesiegerät, umfassend eine Steuerung (400), eine Atemleitung (200), einen handgesteuerten Zweig (500), einen maschinengesteuerten Zweig (300) und das Dreiwegeventil (100) gemäß einem der Ansprüche 1 bis 14, wobei der erste Kanal (111) mit der Atemleitung (200) verbunden ist, der zweite Kanal (112) mit dem maschinengesteuerten Zweig (300), der dritte Kanal (113) mit dem handgesteuerten Zweig (500) und die Steuerung (400) elektrisch mit dem elektrischen Schaltmechanismus (2) verbunden ist, um das Dreiwegeventil (100) so zu steuern, dass es wahlweise den maschinengesteuerten Zweig (300) mit der Atemleitung (200) oder den handgesteuerten Zweig (500) mit der Atemleitung (200) verbindet.

## Revendications

1. Vanne à trois voies (100), comprenant :
un corps de vanne à trois voies (11), où le corps de vanne à trois voies (11) est doté d'une cavité de coulissement (110), et d'un premier canal (111), d'un deuxième canal (112) et d'un troisième canal (113) lesquels sont respectivement reliés à la cavité de coulissement (110) ;
un ensemble de noyau de vanne (12) lequel est agencé à l'intérieur du corps de vanne à trois voies (11), où l'ensemble de noyau de vanne (12) comprend une tige de vanne (121) et une partie d'étanchéité (122) laquelle est agencée au niveau de la tige de vanne (121), la tige de vanne (121) est configurée pour amener la partie d'étanchéité (122) à coulisser à l'intérieur de la cavité de coulissement (110) d'une manière en va-et-vient ; où l'ensemble de noyau de vanne (12) comprend en outre une première position de fonctionnement et une seconde position de fonctionnement, lorsque l'ensemble de noyau de vanne (12) se trouve à la première position de fonctionnement, la partie d'étanchéité (122) ferme le deuxième canal (112), et le premier canal (111) est relié au troisième canal (113), lorsque l'ensemble de noyau de vanne (12) se trouve à la seconde position de fonctionnement, la partie d'étanchéité (122) ferme le troisième canal (113), et le premier canal (111) est relié au deuxième canal (112) ; et
un mécanisme de commutation électrique (2) lequel est au moins en partie agencé à l'extérieur du corps de vanne à trois voies (11) et comprend une partie d'alimentation (2') et un élément mobile (2") lesquels sont en connexion d'entraînement, où le mécanisme de commutation électrique (2) est configuré pour amener la tige de vanne (121) à se déplacer afin d'amener la partie d'étanchéité (122) à coulisser à l'intérieur de la cavité de coulissement (110) ;
où la tige de vanne (121) est en mesure de s'étendre jusqu'à une surface extérieure du corps de vanne à trois voies (11) ou de s'étendre à l'extérieur du corps de vanne à trois voies (11), et l'élément mobile (2") du mécanisme de commutation électrique (2) et la tige de vanne (121) sont sélectivement séparés ou en connexion d'entraînement, pour pousser la tige de vanne (121) pour la coulisser dans un premier sens, où la vanne à trois voies (100) comprend en outre un élément élastique (14) lequel est en connexion d'entraînement avec l'ensemble de noyau de vanne (12) pour exercer une force dans un sens opposé de l'élément mobile (2"), pour amener l'ensemble de noyau de vanne (12) à se repositionner ; et
où la partie d'alimentation (2') du mécanisme de commutation électrique (2) comprend un élément d'entraînement cylindrique (21) et une électrovanne pilote (22), l'élément mobile (2") est une tige de piston (212), l'élément d'entraînement cylindrique (21) comprend une base de vanne (210) et un piston (211) lequel est relié de manière fixe à la tige de piston (212), la base de vanne (210) est dotée d'une cavité de vanne (2101) et d'une première entrée de gaz laquelle est reliée à la cavité de vanne (2101), et où l'électrovanne pilote (22) est configurée pour relier la première entrée de gaz à une source de gaz, de sorte que la tige de piston (212) est en mesure d'amener la tige de vanne (121) à se déplacer.

2. Vanne à trois voies (100) selon la revendication 1, dans laquelle une seconde extrémité de la tige de vanne (121) s'étend jusque dans le deuxième canal (112), et le deuxième canal (112) est dotée d'une butée (112a) et une première extrémité de l'élément élastique (14) vient buter contre la butée (112a) ;
une seconde extrémité de l'élément élastique (14) vient buter contre la partie d'étanchéité (122), ou une structure de collerette (1211) est formée au niveau de la tige de vanne (121) et une seconde extrémité de l'élément élastique (14) vient buter contre la structure de collerette (1211).

3. Vanne à trois voies (100) selon la revendication 1, où la vanne à trois voies (100) comprend en outre une première partie de guidage (131) laquelle est agencée à l'intérieur du deuxième canal (112) ; où la première partie de guidage (131) est dotée d'une première rainure de coulissement (131a) laquelle s'étend le long d'une direction longitudinale de la tige de vanne (121), et une seconde extrémité de la tige de vanne (121) pénètre jusque dans et est agencée avec possibilité de coulissement à l'intérieur de la première rainure de coulissement (131a).

4. Vanne à trois voies (100) selon la revendication 3, dans laquelle la seconde extrémité de la tige de vanne (121) comprend un premier segment (1212) et un second segment (1213) lesquels sont contigus ; où un diamètre extérieur du premier segment (1212) est inférieur à un diamètre extérieur du second segment (1213), de telle sorte qu'une surface d'épaulement (121a) est formée au niveau d'une jonction du second segment (1213) et du premier segment (1212) ; où le premier segment (1212) pénètre jusque dans et est agencé avec possibilité de coulissement à l'intérieur de la première rainure de coulissement (131a), et un côté de la première partie de guidage (131), qui fait face à la partie d'étanchéité (122), vient buter contre la surface d'épaulement (121a).

5. Vanne à trois voies (100) selon la revendication 1, où
la vanne à trois voies (100) comprend en outre une seconde partie de guidage (132) laquelle est agencée à l'intérieur du corps de vanne à trois voies (11) ; où la seconde partie de guidage (132) est dotée d'une seconde rainure de coulissement (132a) laquelle s'étend à l'extérieur du corps de vanne à trois voies (11) le long d'une direction longitudinale de la tige de vanne (121), où une première extrémité de la tige de vanne (121) pénètre jusque dans et est agencée avec possibilité de coulissement et hermétiquement à l'intérieur de la seconde rainure de coulissement (132a).

6. Vanne à trois voies (100) selon la revendication 1, dans laquelle la partie d'étanchéité (122) est une plaque de vanne, où une jonction de la cavité de coulissement (110) et du deuxième canal (112) est dotée d'une première surface d'étanchéité annulaire (110a), et la plaque de vanne est configurée pour venir buter hermétiquement contre la première surface d'étanchéité annulaire (110a) pour fermer le deuxième canal (112) ; et/ou une jonction de la cavité de coulissement (110) et du troisième canal (113) est dotée d'une seconde surface d'étanchéité annulaire (110b), et la plaque de vanne est configurée pour venir buter hermétiquement contre la seconde surface d'étanchéité annulaire (110b) pour fermer le troisième canal (113).

7. Vanne à trois voies (100) selon la revendication 6, dans laquelle lorsque l'ensemble de noyau de vanne (12) se trouve à la première position de fonctionnement, une partie de bord de la plaque de vanne le long d'un côté axial de la tige de vanne (121) vient buter hermétiquement contre la première surface d'étanchéité annulaire (110a), et lorsque l'ensemble de noyau de vanne (12) se trouve à la seconde position de fonctionnement, une partie de bord de la plaque de vanne le long d'un côté axial opposé de la tige de vanne (121) vient buter hermétiquement contre la seconde surface d'étanchéité annulaire (110b).

8. Vanne à trois voies (100) selon la revendication 1, où la vanne à trois voies (100) comprend en outre un dispositif de détection de position (16) lequel est configuré pour détecter une position de fonctionnement de l'ensemble de noyau de vanne (12) ; où lorsque l'ensemble de noyau de vanne (12) se trouve à la première position de fonctionnement, le dispositif de détection de position (16) est configuré pour déclencher et générer un premier signal de position ; et/ou lorsque l'ensemble de noyau de vanne (12) se trouve à la seconde position de fonctionnement, le dispositif de détection de position (16) est configuré pour déclencher et générer un second signal de position.

9. Vanne à trois voies (100) selon la revendication 8, dans laquelle le dispositif de détection de position (16) comprend un premier commutateur de déplacement (161) et un second commutateur de déplacement (162), la vanne à trois voies (100) comprend un ensemble de bascule (15), où la tige de vanne (121) est en connexion d'entraînement avec l'ensemble de bascule (15) pour amener sélectivement l'ensemble de bascule (15) à déclencher le premier commutateur de déplacement (161) ou le second commutateur de déplacement (162).

10. Vanne à trois voies (100) selon la revendication 9, dans laquelle l'ensemble de bascule (15) comprend une tige de bascule (151) et une plaque de bascule (152), la plaque de bascule (152) tourne par rapport au corps de vanne à trois voies (11), la tige de bascule (151) est reliée de manière fixe à une première extrémité de la tige de vanne (121) ; où la tige de bascule (151) est configurée pour amener la plaque de bascule (152) à tourner vers l'avant ou l'arrière durant une mouvement de va-et-vient linéaire suivant la tige de vanne (121), de sorte à déclencher sélectivement le premier commutateur de déplacement (161) ou le second commutateur de déplacement (162) ; de préférence, un espace d'installation (114) est formé à l'extérieur du corps de vanne à trois voies (11), et la tige de bascule (151) est agencée à l'intérieur de l'espace d'installation (114).

11. Vanne à trois voies (100) selon la revendication 1, dans laquelle la partie d'alimentation (2') amène l'élément mobile (2") à coulisser d'une manière en va-et-vient ou à tourner, et le mécanisme de commutation électrique (2) amène la tige de vanne (121) à coulisser par l'intermédiaire de l'élément mobile (2").

12. Vanne à trois voies (100) selon la revendication 1, dans laquelle la cavité avec tige (2101a) est reliée à l'atmosphère, l'élément d'entraînement cylindrique (21) comprend en outre un ressort de rappel (213), lequel est emmanché au niveau de la tige de piston (212) et est configuré pour amener la tige de piston (212) à se repositionner.

13. Vanne à trois voies (100) selon la revendication 11, dans laquelle le mécanisme de commutation électrique (2) est un moteur électrique linéaire, la partie d'alimentation (2') est un stator du moteur électrique linéaire, et l'élément mobile (2") est un rotor du moteur électrique linéaire.

14. Vanne à trois voies (100) selon la revendication 1, dans laquelle le deuxième canal (112) s'étend approximativement le long d'une ligne droite, la cavité de coulissement (110) s'étend approximativement le long d'une ligne droite, et la cavité de coulissement (10) et le deuxième canal (112) sont situés sur la même ligne droite.

15. Machine d'anesthésie, comprenant une unité de commande (400), une ligne respiratoire (200), une branche à commande manuelle (500), une branche à commande par machine (300) et la vanne à trois voies (100) selon l'une quelconque des revendications 1 à 14, dans laquelle le premier canal (111) est relié à la ligne respiratoire (200), le deuxième canal (112) est relié à la branche à commande par machine (300), le troisième canal (113) est relié à la branche à commande manuelle (500), et l'unité de commande (400) est électriquement reliée au mécanisme de commutation électrique (2) pour commander la vanne à trois voies (100) pour sélectivement relier la branche à commande par machine (300) à la ligne respiratoire (200) ou relier la branche à commande manuelle (500) à la ligne respiratoire (200).
